# EUROPÄISCHE PATENTSCHRIFT

(11) **EP 0 866 256 B1**
(45) Veröffentlichungstag und Bekanntmachung des Hinweises auf die Patenterteilung: **11.07.2001**
(21) Anmeldenummer: 98104009.0
(22) Anmeldetag: 06.03.1998
(51) Int. Cl.: F16L 33/22, F16L 47/04, F16L 19/02, A61M 39/12

(54) **Klemmverbinder**
Clamping connection
Accouplement de serrage

(30) Priorität: 22.03.1997 DE 19712171
(43) Veröffentlichungstag der Anmeldung: 23.09.1998
(73) Patentinhaber: PRIMED HALBERSTADT MEDIZINTECHNIK GMBH, 38820 Halberstadt (DE)
(72) Erfinder: Maltzahn, Meik, 38820 Halberstadt (DE); Neubauer, Norbert, 38820 Halberstadt (DE)
(74) Vertreter: Pfeiffer, Rolf-Gerd, Dipl.-Phys.

(56) Entgegenhaltungen:
- EP-A- 0 645 161
- DE-A- 3 503 562
- DE-A- 3 624 745
- FR-A- 2 172 310
- FR-A- 2 436 321

## Beschreibung

Die Erfindung betrifft einen Klemmverbinder zur dichtenden und fixierenden Verbindung eines Rohres oder Schlauches, insbesondere eines Drains, der in der Medizin Verwendung findet, mit einem schlauchartigen Abfluß.

Nach dem bislang bekannten Stand der Technik werden zur Verbindung eines Drains mit einem Schlauch im Rahmen einer aktiven Wunddrainage zum einen Stufenverbinder verschiedener Hersteller eingesetzt, die innerhalb eines Kopplungsteils mehrere abgestufte Durchmesserbereiche zur Aufnahme eines dem jeweiligen Innendurchmesser entsprechenden Draindurchmessers aufweisen. Je nach eingesetztem Draindurchmesser ist der Stufenverbinder soweit abzuschneiden, bis der gewünschte Aufnahmedurchmesser freiliegt. Am anderen Ende des Stufenverbinders wird ein Verbindungsschlauch zu weiteren Baugruppen, wie Faltenbälgen o.ä. in den Stufenverbinder eingesteckt. Diese Stufenverbinder bergen Risiken bezüglich der Drainhaltekraft und der Dichtheit der Koppelverbindung in sich. Abgesehen davon, stellt das erforderliche Abschneiden des Stufenverbinders auf den gewünschten Durchmesserbereich einen zusätzlichen, in der medizinischen Praxis unerwünschten Arbeitsschritt dar. Weiterhin sind bei dieser Bauform auch nur abgestufte, den Stufen des Verbinders entsprechende Draindurchmesser einsetzbar.
Zum anderen sind für den gleichen Verwendungszweck Drehverbinder unterschiedlicher Hersteller auf dem Markt, die aus einer mit einem Außengewinde versehenen Hülse bestehen, in die an einem Ende ein Verbindungsschlauch eingeklebt ist. In die Hülse ist lose ein elastisches Schlauchstück eingelegt. Das mit dieser Hülse in Verbindung zu bringende Drain wird durch ein überwurfmutterartiges Teil eingefädelt und in das elastische Schlauchstück eingeschoben. Anschließend wird das überwurfmutterartige Teil mit dem Außengewinde der Hülse in Eingriff gebracht und solange gegen das Außengewinde verdreht, bis ein in der Hülse vorgesehener Zylinder das elastische Schlauchstück zusammenquetscht, wobei sich dieser in undefinierter Weise an das Drain anschmiegt. Diese Art der Verbindung bedingt ein recht kompliziertes Einlegen und Fixieren des Drains und je nach individuellen Gefühl der Anwender sehr unterschiedliche Haltekräfte, da das Drehmoment in der Endstellung keiner Kontrolle unterliegt. Beim Einsatz dieser Verbindungen entstehen häufig undichte Drainanschlüsse oder es kommt zum Abquetschen des Drains selbst. In FR 2,172,310 ist eine Kupplung zum Anschluß an ein Rohrstück oder zum koaxialen Verbinden zweier ausgerichteter Rohre bekannt, bei der ein Rastgesperre zum Einsatz gelangt. Neben der ebenfalls zu komplizierten Handhabung dieser Vorrichtung für den hier vorgesehenen Verwendungszweck besitzt diese Vorrichtung zusätzlich den Nachteil, daß das dort umlaufend ausgeführte Rastgesperre nur mit einem relativ großem Kraftaufwand lösbar ist. Weitere Quetschverbinder, denen ein oder mehrere der oben beschriebenen Nachteile anhaften, sind bspw. in DE-AS 1 207 736, DE 35 03 562 A1, DE 21 06 894 A1, DE 96 16 973 U1, DE 82 14 948 U1 und DE 1 675 346 A1 beschrieben.

Der Erfindung liegt die Aufgabe zugrunde, einen Klemmverbinder zu schaffen, der leicht handhabbar ist und unabhängig vom Bedienpersonal eine dichtenden Verbindung eines Drains mit weitestgehend definierten Haltekräften gewährleistet und die Nachteile des Standes der Technik vermeidet.

Die Aufgabe wird durch die kennzeichnenden Merkmale des ersten Patentanspruchs gelöst. Vorteilhafte Ausgestaltungen sind durch die nachgeordneten Ansprüche erfaßt.
Das Wesen der Erfindung besteht darin, daß zwei vorgesehene Bauteile miteinander in eine unverlierbare Verbindung gebracht sind, diese Bauteile gegeneinander wenigstens um 90° verdrehbar sind und wenigstens zwei Verdrehpositionen vorgesehen sind, bei denen erfindungsgemäß vorgesehene Rastmittel ineinandergreifen bzw. zumindest teilweise außer Eingriff bringbar sind, und daß eine im Rastabstand der Rastmittel definierte Druckbeaufschlagung eines elastischen Dichtmittels bewirkt wird.

Die Erfindung soll nachstehend anhand eines Ausführungsbeispiels näher erläutert werden. Es zeigen:
- Fig. 1: einen Stufenverbinder nach dem Stand der Technik,
- Fig. 2: einen Drehverbinder nach dem Stand der Technik,
- Fig. 3: eine Ausführungsform eines Klemmverbinders nach vorliegender Erfindung in einem seitlichen Längsschnitt,
- Fig. 3a: eine Innenansicht einer Fixierkappe als Teil des Klemmverbinders und
- Fig. 3b: eine seitliche Draufsicht auf einen ersten Teil des Klemmverbinders nach Fig. 3.

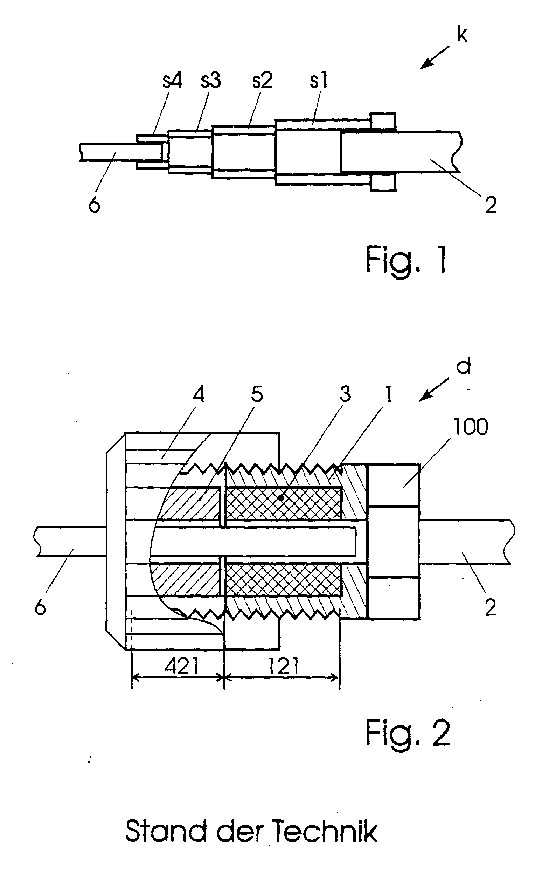

In Figur 1 ist ein nach dem Stand der Technik bekannter Stufenverbinder k dargestellt, der im Beispiel vier Stufungen s1 bis s4 mit sich stufig verjüngenden Innendurchmessern besitzt. Ein dem Innendurchmesser der Stufe s4 im Außendurchmesser entsprechendes Drain 6 ist klemmend in die Stufe s4 eingesteckt. Soll ein Drain mit einem vergrößerten Außendurchmesser eingesetzt werden, der bspw. dem Innendurchmesser der Stufe s3 entspricht, so ist die gesamte Stufe s4 abzuschneiden. An dem der Drainaufnahme gegenüberliegenden Ende ist der Stufenverbinder k mit einem zu weiteren Baugruppen einer aktiven Wunddrainage führenden Schlauchabfluß 2 versehen. Diese Bauform weist die im zitierten Stand der Technik aufgeführten Mängel auf.

Figur 2 zeigt einen weiteren nach dem Stand der Technik bekannten Drehverbinder d in einer teilweise geschnittenen Darstellung. Dieser Drehverbinder d besitzt eine hülsenartige Baugruppe 1, die mit einem flügelmutterartigen Halteteil 100 verbunden ist und in welche ein Schlauchabfluß 2 eingeklebt ist. Innerhalb der Baugruppe 1 ist ein elastisches Schlauchstück 3 eingelegt. am äußeren Umfang trägt die Baugruppe 1 einen in sich geschlossenen Gewindegang 121, in den ein entsprechendes Gegengewinde 423 eines zweiten überwurfmutterartigen Bauteils 4 eingreifen kann. Bevor dieses Bauteil 4 auf die Baugruppe 1 aufgesetzt wird, ist ein Drain 6 durch eine mittige Öffnung des Bauteils 4 zu fädeln und in den Bereich des elastischen Schlauchstücks 3, wie in Fig. 2 dargestellt, einzuführen und zu positionieren. Anschließend erfolgt ein Verschrauben der Teile 1 und 4 gegeneinander, wobei ein Hohlzylinder 5 mit fortschreitender Verschraubung auf das elastische Schlauchstück 3 drückt und dieses zusammenquetscht. Neben den hierzu im Stand der Technik aufgeführten Mängeln, kann es insbesondere beim Einsatz von Drains 6 mit kleinem Außendurchmesser zusätzlich zu einem Insichverwinden des Schlauchstücks 3 kommen, was ein Abquetschen des Drains zur Folge haben kann.

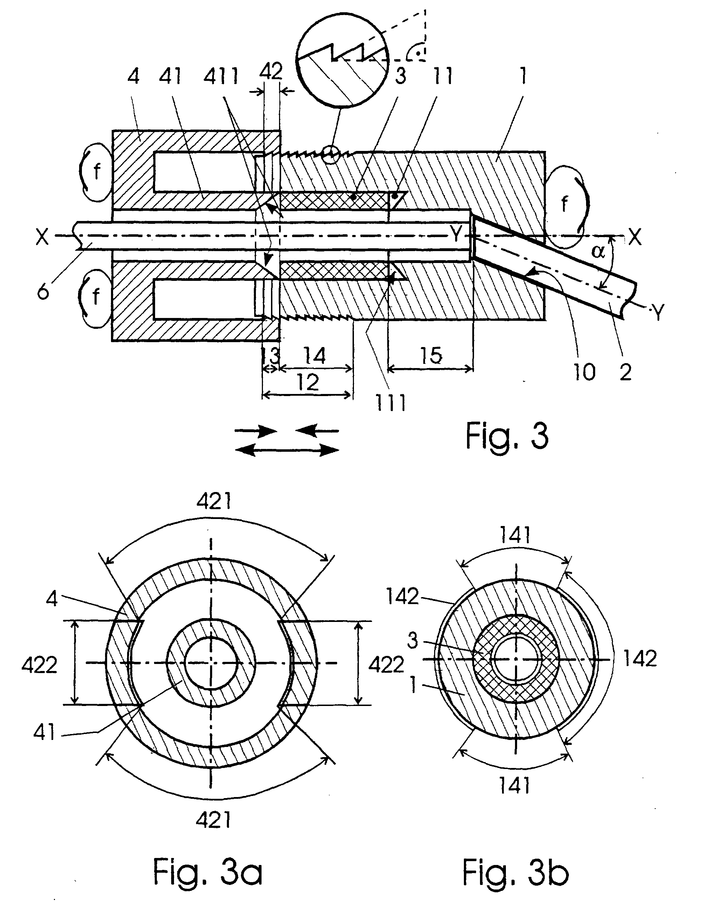

In Figur 3 ist schließlich eine Ausführung eines Klemmverbinders gemäß vorliegender Erfindung dargestellt. Dieser Klemmverbinder besteht aus einem ersten Bauteil 1, in das eine Ausnehmung 10 derart eingebracht ist, daß die Achse Y-Y der Ausnehmung 10 auf der Achse X-X des Bauteils 1 ausläuft, wobei die Achse Y-Y mit der Achse X -X des Bauteils 1 einen Winkel α, im Beispiel α ≈ 30°, einschließt. Die Festlegung des Winkels α erfolgt mit der Maßgabe, daß er groß festgelegt ist, daß die Randbereiche der Ausnehmung (10) außerhalb des von der Achse (X-X) durchstoßenen Bereiches liegen. In die Ausnehmung 10 ist ein Schlauchende 2 eingebracht und mit der Ausnehmung 10 verklebt. Weiterhin ist im Bauteil 1 eine stufenförmige Ausnehmung 11 vorgesehen, die der Aufnahme eines elastischen Schlauchstücks 3 dient. Das Bauteil 1 ist in einem dem Schlauchanschluß 2 gegenüberliegenden Außenumfangsbereich mit einem ersten Rastgesperre 12 versehen, das umfangsmäßig in zwei Teilbereiche 13 und 14 unterteilt ist. Dabei ist das Rastgesperre 12 im Bereich 13 umfangsmäßig geschlossen ausgebildet, wohingegen ihm im Bereich 14 parallel zur Achse X-X wenigstens zwei unterbrochene, einander gegenüberliegende Bereiche 141 (vgl. Fig. 3b) gegeben sind, deren Funktion weiter unten beschrieben wird. Weiterhin besitzt der Klemmverbinder eine Fixierkappe 4, der einige Gänge eines Gegenrastgesperres 42 gegeben sind, welche rastend in das Rastgesperre 12 eingreifen und soweit in der Vormontage des Klemmverbinders auf das Teil 1 aufgedrückt wird, daß sie die zwei bis drei umfangsmäßig geschlossenen Gesperregänge im Bereich 13 überspringen. Damit ist die Fixierkappe 4 unverlierbar mit dem Teil 1 verbunden. Wesentlich ist, daß das Gegenrastgesperre 42 unterbrochen ausgeführt ist und (vgl. Fig. 3a) senkrecht und parallel zur Zeichenebene Bereiche 421 aufweist, die kein Gegenrastgesperre tragen. Anschließend erfolgt die Einführung eines Drains 6, wie in Fig. 3 dargestellt. Da zumindest das Bauteil 1 aus einem durchsichtigen Material gefertigt sein soll und die schräge Herausführung der Ausnehmung 10 einen gewissen Widerstand beim Einführen des Drains 6 bildet, kann die Positionierung des Drains 6 innerhalb der Klemmvorrichtung problemlos ermittelt werden, zumal ein Beobachtungsbereich 15 vorgesehen ist. Das medizinische Personal kann jetzt mit drei Fingern f einer Hand die Fixierkappe 4, entsprechend der unter Fig. 3 angedeuteten, gegeneinander gerichteten Pfeile, soweit gegen das Teil 1 stufenweise zusammendrücken, bis das elastische Schlauchstück 3 klemmend und dichtend am Drain 6 anliegt, wobei ein rastender Eingriff der Rastgesperrebereiche 42 (422) und 14 (142) gegeneinander erfolgt. Die Klemm- und Dichtwirkung des elastischen Schlauchstücks 3 wird dabei durch die Einwirkung eines Hohlzylinders 41, der bevorzugt ein einstückiges Bestandteil der Fixierkappe 4 ist, erreicht. Der Hohlzylinder 41 ist an seinem dem Schlauchstück 3 zugewandten Ende mit einer Zentrierfase 411, die nach außen radial ansteigend ausgeführt ist, versehen, wohingegen die andere Anlage des Schlauchstücks 3 in dem Teil 1 bevorzugt mit einer radial nach außen abfallenden Zentrierfase 111 versehen ist, wodurch eine sichere Abdichtung und Lagefixierung des Schlauchstücks 3 während des Zusammendrückens der Bauteile 1 und 4 erreicht gewährleistet ist. Wie bereits ausgeführt, ist das Rastgesperre 12 im Bereich 13 umfangsmäßig durchgehend geschlossen ausgeführt, während es im Bereich 14 unterbrochen ausgeführt ist. Letzteres verdeutlicht Fig. 3b, die die verbleibend vorgesehenen Rastbereiche 142 anzeigt; die Rastbereiche der Zone 13 sind in Fig. 3b aus Übersichtlichkeitsgründen nicht dargestellt. Durch eine Verdrehung der Fixierkappe 4 soweit, daß die Rastbereiche 421 mit den Bereichen 141 des Teils 1 korrespondieren, ist der rastende Eingriff aufhebbar, die Fixierkappe 4 kann bis zum Bereich 13, vgl. Doppelpfeil in Fig. 3, verschoben und das Drain 6 entfernt werden. Dieser Klemm- und Lösevorgang ist beliebig oft wiederholbar und eine Dekonnektierung vom patientenseitig angelegten Drain ist jederzeit möglich. Infolge des im Bereich 13 vorgesehenen umfangsmäßig geschlossenen Rastgesperres 12, verbleibt die Fixierkappe unverlierbar am Bauteil 1. Um die Verschiebe- bzw. Rastwirkung fördernd zu begünstigen sind die sägezahnartigen Rastmittel 12 und 42 im Schnitt in Form ungleichseitiger, rechwinkliger und ineinandergreifender Dreiecke ausgebildet, wie es die entsprechende Vergrößerung in Fig. 3 andeutet. Bei einer erneuten Klemmung des Drains 6 braucht die Fixierkappe 4 lediglich entgegen obiger Drehrichtung verdreht werden und gelangt so wieder in rastenden Eingriff.

Der erfindungsgemäße Klemmverbinder ist nicht auf die beschriebene bevorzugte Anwendung im medizinischen Bereich beschränkt; er kann vorteilhaft überall dort, wo einseitig verlegte Schläuche (hier Drain), Rohre oder Stäbe, insbesondere mit variierenden Durchmessern, mit weiteren Baugruppen zu verbinden sind, eingesetzt werden.

Alle in der Beschreibung, den nachfolgenden Ansprüchen und der Zeichnung dargestellten Merkmale können sowohl einzeln als auch in beliebiger Kombination miteinander erfindungswesentlich sein.

### Bezugszeichenliste

- 1 -: hülsenartiges erstes Bauteil
- 10 -: Ausnehmung zur Aufnahme des Schlauches 2
- 11 -: stufenartige Ausnehmung des Bauteils 1
- 111 -: Zentrierfase der Aufnehmung 11
- 12-: Außenumfangsbereich des Teils 1 mit Rastgesperre
- 13-: Teilbereich von 12 mit umfangsmäßig geschlossenem Rastgesperre
- 14, 141 -: Teilbereich von 12 mit umfangsmäßig unterbrochenem Rastgesperre
- 142 -: Teilbereich von 12 mit Rastgesperre
- 15 -: durchsichtiger Bereich des Teils 1
- 2 -: Schlauch
- 3 -: elastisches Schlauchstück
- 4 -: Teil 1 umfassendes zweites Bauteil / Fixierungskappe
- 41 -: Hohlzylinder
- 411 -: Zentrierungsfase des Hohlzylinders
- 42 -: Gegenrastgesperre
- 421 -: unterbrochene Bereiche des Rastgesperres 42
- 422 -: Bereiche des Gegenrastgesperres 42 mit Rastgesperre
- 5 -: Hohlzylinder
- 6 -: zweiter Schlauch (Drain)
- d -: Drehverbinder
- 100 -: flügelmutterartiges Halteteil
- 121 -: geschlossener Gewindegang
- 423 -: geschlossener Gewindegang (Stand der Technik)
- k -: Stufenverbinder
- sl-s4 -: Stufungen des Verbinders k
- X-X, Y-Y -: Achsen
- α: Winkel zwischen den AchsenX-X und Y-Y

## Patentansprüche

1. Klemmverbinder bestehend aus einem hülsenartigen ersten Bauteil (1), das an einem ersten Ende mit einem Schlauch oder Rohr (2) versehbar und in das ein elastisches Schlauchstück (3) eingelegt ist und einem zweiten überwurfmutterartigen, das erste Bauteil (1) umfassenden zweiten Bauteil, das als Fixierungskappe (4) ausgebildet und mit dem ersten Bauteil (1) verbindbar ist, und innwändig einen das elastische Schlauchstück (3) erfassenden Hohlzylinder trägt, durch den ein zweiter Schlauch oder ein zweites Rohr (6) einführbar ist, wobei das elastische Schlauchstück (3) in eine stufenartige Ausnehmung (11) des ersten Bauteils (1) eingelegt ist, das erste Bauteil (1) in einem dem ersten Schlauch (2) gegenüberliegenden Außenumfangsbereich mit einem ersten Rastgesperre (12) versehen ist, in das ein entsprechend angepaßtes Gegenrastgesperre (42) der Fixierungskappe (4) eingreift, und daß der Schlauch (2) am ersten Ende des Bauteils (1) in eine Ausnehmung (10) eingebracht ist, deren Achse (Y-Y) auf der Achse (X-X) endet und mit dieser einen Winkel α einschließt, der so groß festgelegt ist, daß die Randbereiche der Ausnehmung (10) außerhalb des von der Achse (X-X) durchstoßenen Bereiches liegen.

2. Klemmverbinder nach Anspruch 1, dadurch gekennzeichnet, daß das Rastgesperre (12) des Teils (1) in einem Bereich (14; 141) unterbrochen und im einem Endbereich (13) umfangsmäßig in sich geschlossen ausgebildet ist.

3. Klemmverbinder nach Anspruch 1 und 2, dadurch gekennzeichnet, daß das Rastgesperre (42) der Fixierungskappe (4) unterbrochen ausgebildet ist, wobei die unterbrochenen Bereiche (421) umfangsmäßig mindestens so groß wie die im Bereich (14) des Teils (1) verbliebenen Rastbereiche (142) festgelegt sind.

4. Klemmverbinder nach Anspruch 1, dadurch gekennzeichnet, daß *der* innwändige Hohlzylinder (41) der Fixierungskappe (4) mit einer das elastische Schlauchstück (3) erfassenden Zentrierungsfase (411) versehen ist, die radial nach außen ansteigend festgelegt ist.

5. Klemmverbinder nach Anspruch 1, dadurch gekennzeichnet, daß die Bodenseite der stufenförmigen Ausnehmung (11) mit einer radial nach außen abfallenden Zentrierfase (111) versehen ist.

6. Klemmverbinder nach einem der vorstehenden Ansprüche, dadurch gekennzeichnet, daß das Teil (1) und die Fixierkappe (4) jeweils als einstückige Spritzteile gefertigt sind.

7. Klemmverbinder nach Anspruch 6, dadurch gekennzeichnet, daß das zumindest für das Material des Teiles (1) ein durchsichtiger Kunststoff gewählt ist und zumindest in einem Bereich (15) ein Sichtfenster verbleibt.

## Claims

1. Clamping connector comprising a shell-shaped first unit (1) and a second cap-screw like unit, said first unit (1), into which an elastic hose piece (3) is inserted, is adapted to be provided, via a first end portion, with a hose or a tube (2), said second unit being designed as a fixing cap (4) encompassing said first unit (1) and being connectable to said first unit (1), said second unit being internally provided with a hollow cylinder which is adapted for capturing said elastic hose piece (3), a second hose or a second tube (6) are adapted for being inserted through said hollow cylinder, whereby said elastic hose piece (3) being fitted into a step-like lowered recess (11) of said first unit (1), said first unit (1) being provided with a first arresting ratchet (12), said arresting ratchet being provided on a circumferential range remote from said first hose (2), a counter arresting ratchet (42) of said fixing cap (4) being correspondingly adapted to and meshing said first arresting ratchet (12), and in that said hose (2) is inserted into a passage (10) provided in said first end portion of the unit (1), the axis (Y- Y) of said passage ends on and includes an angle α; with the axis (X - X), said angle α; being selected so great that the rim portions of the passage (10) lie outside of the range being passed through by the axis (X- X).

2. Clamping connector as claimed in claim 1, characterized in that the arresting ratchet (12) of the unit (1) being designed as interrupted in the range (14; 141) and as being circumferentially closed in itself in an end portion (13).

3. Clamping connector as claimed in claims 1 and 2, characterized in that the arresting ratchet (42) of said fixing cap (4) being designed as interrupted, the interrupted ranges (421) being circumferentially dimensioned at least as great as the arresting ranges (142) remaining in the range (14) of the unit (1).

4. Clamping connector as claimed in claim 1, characterized in that the internal hollow cylinder (41) of the fixing cap (4) is provided with a centring bevel (411) engaging said elastic hose piece (3), said bevel being designed as radially rising towards outside.

5. Clamping connector as claimed in claim 1, characterized in that the bottom side of the step-like recess (11) is provided with a centring bevel (111) radially descending towards outside.

6. Clamping connector as claimed in any of the preceding claims, characterized in that the unit (1) and the fixing cap (4) are manufactured by injection moulding in one piece each.

7. Clamping connector as claimed in claim 6, characterized in that a transparent plastic is selected at least for the material of the unit (1) and in that a viewing window remains at least in a range (15).

## Revendications

1. L'accouplement de serrage est composé d'un premier élément en forme de douille (1), dont une extrémité est dotée d'un tuyau flexible ou d'un tube (2) dans lequel une partie d'un tuyau flexible (3) est insérée, et d'un deuxième élément en forme d'écrou-raccord enveloppant le premier élément, lequel étant conçu comme capuchon de fixation (4) à relier avec le premier élément (1) et pourvu à l'intérieur d'un cylindre creux dans lequel est placé le tuyau flexible (3) et à travers lequel il est possible d'introduire un deuxième tuyau flexible ou un deuxième tube (6), le tuyau flexible étant (3) logé à l'intérieur du premier élément (1) dans un évidement en forme de gradin, le premier élément (1) étant doté d'un premier dispositif d'encliquetage (12) monté sur la surface extérieur de l'élément en face du premier tuyau flexible, dispositif dans lequel s'enclique le dispositif de blocage (42) placé sur le capuchon de fixation (4), le tuyau flexible (2) placé sur la première extrémité (1) étant logé dans un évidement (10) dont l'axe (Y-Y) débouche sur l'axe (X-X) avec lequel il forme un angle α défini de sorte que les bords de l'évidement (10) se trouvent situés à l'extérieur de la zone traversée par l'axe (X -X).

2. L'accouplement de serrage conçu selon la revendication 1 est caractérisé en ce que le dispositif d'encliquetage (12) de l'élément (1) est interrompu dans la zone ( 14; 141) et que son pourtour est fermé aux bords de la zone (13).

3. L'accouplement de serrage conçu selon les revendications 1 et 2 est caractérisé en ce que le dispositif d'encliquetage (42) du capuchon (4) est conçu de manière interrompue, les pourtours des zones interrompues (421) étant au moins aussi grands que les zones d'encliquetage (142) recouvertes par la zone (14) de l'élément (1).

4. L'accouplement de serrage conçu selon la revendication 1 est caractérisé en ce que le cylindre creux (41) situé sur la paroi intérieure (41) du capuchon de fixation (4) est pourvu d'un chanfrein de centrage (411) présentant une forme ascendante vers l'extérieur.

5. L'accouplement de serrage conçu selon la revendication 1 est caractérisé en ce que le fond de l'évidement à gradin (11) est pourvu d'un chanfrein de centrage (111) présentant une forme redescendant vers l'extérieur.

6. L'accouplement de serrage conçu selon une des revendications précédentes est caractérisé en ce que l'élément (1) et le capuchon de fixation (4 ) sont des composants formant chacun une seule pièce produite par injection.

7. L'accouplement de serrage conçu selon la revendication 6 est caractérisé en ce qu'au moins l'élément (1) est réalisé dans une matière plastique transparente et qu'il existe un voyant de contrôle sur une partie du dispositif (15).
